# EUROPEAN PATENT APPLICATION

(11) **EP 4 502 146 A1**
(43) Date of publication of application: **05.02.2025**
(21) Application number: 23780457.0
(22) Date of filing: 28.03.2023
(51) Int. Cl.: C12N 5/0775, C12M 3/00, C12N 5/071

(54) **METHOD FOR CULTIVATING MESENCHYMAL STEM CELL**

(30) Priority: 29.03.2022 JP 2022054023
(71) Applicant: National University Corporation Tokai National Higher Education and Research System, Nagoya-shi, Aichi 464-8601 (JP); Nipro Corporation, Osaka 566-8510 (JP)
(72) Inventor: FURUHASHI, Kazuhiro, Nagoya-shi, Aichi 464-8601 (JP); MARUYAMA, Shoichi, Nagoya-shi, Aichi 464-8601 (JP); TANAKA, Akihito, Nagoya-shi, Aichi 464-8601 (JP); TAKASU, Masaki, Nagoya-shi, Aichi 464-8601 (JP); SUNOHARA, Takashi, Settsu-shi, Osaka 5668510 (JP); YAMAGUCHI, Satoru, Settsu-shi, Osaka 5668510 (JP)
(74) Representative: Novagraaf International SA
(86) International application number: PCT/JP2023/012386
(87) International publication number: WO 2023/190448

(57) **Abstract**

A method for efficiently culturing mesenchymal stem cells (MSCs) on hollow fiber membranes is provided. Another object is to provide a means for applying cultured MSCs on hollow fiber membranes to diseases.

MSCs are cultured by a method that includes: a step of hydrophilizing the outer luminal surface of a hollow fiber membrane; a step of placing the hydrophilized hollow fiber membrane in a culture solution for seeding MSCs on the outer luminal surface; and a step of culturing MSCs under conditions of applying hydrodynamic loading into the hollow fiber membrane with the cells seeded thereon. A column cartridge containing MSCs and the hollow fiber membrane, wherein the MSCs are adhered in multiple layers to the outer luminal surface of the hollow fiber membrane, is provided for therapeutic devices.

## Description

### Technical Field

The present invention relates to a method and a device for culturing mesenchymal stem cells, a column cartridge containing cells obtained by the culturing, and a method for using the same.

### Background Art

Acute kidney injury (AKI) often occurs as a complication of other diseases, and it is estimated that 30% to 50% of Intensive Care Unit (ICU) patients present with AKI. The mortality rate of ICU patients with AKI tends to be as high as 30% to 50%, and it has been reported that AKI is strongly correlated with life expectancy, with the life expectancy worsening with increasing severity (Non Patent Literature 1 and Non Patent Literature 2). Recently, the number of patients with novel coronavirus infection (COVID-19) caused by novel coronavirus (SARS-CoV-2) infection is increasing worldwide. The high rate of AKI complications in COVID-19 patients significantly increases their mortality. However, no therapeutic method has yet been found to inhibit the progress of AKI or to promote its recovery.

In recent years, various studies have been conducted on the use of mesenchymal stem cells (MSCs) in the treatment of diseases. MSCs are known to be capable of differentiating into cells belonging to the mesenchymal lineage and to be present in almost all organs, including bone marrow, adipose tissue, placental tissue, dental pulp, etc. The immunomodulatory properties of MSCs are considered useful for therapeutic applications because the stronger the inflammation of the disease, the stronger the immunological effects of MSCs, and because MSCs act locally to the inflammation with little immunosuppressive effects on the whole body.

However, because MSCs are relatively large cells, intravenous administration of the cells themselves carries the risk of causing embolization. MSC administration has been studied for therapeutic applications for acute lung injury and acute renal failure and has been found to have efficacy in animal models. Yet, there is skepticism about the clinical efficacy, and from the perspective of the risk of pulmonary cell embolization and renal cell embolization, there is little future potential.

However, components such as liquid factors secreted by MSCs can be expected to be effective in disease treatment. For example, adipose-derived MSCs (ASCs) have been reported to secrete many growth factors and thus to strongly induce anti-inflammatory macrophages (Non Patent Literature 3). Therefore, there is a demand to establish a therapeutic method using MSCs and the factors they secrete as an alternative to cell administration.

In the field of the medical applications of cells and substances they produce, various methods for efficiently culturing cells for the purpose of stable cell supply have been studied. One example thereof that has been proposed involves seeding cells in the lumen of a hollow fiber membrane and then culturing cells therein (e.g., Patent Literature 1).

It has also been proposed to apply hollow fiber membranes with MSCs retained in the lumina as a bioreactor cartridge for the treatment of acute liver failure (Patent Literature 2).

### Citation List

### Patent Literature

Patent Literature 1: Japanese Patent Laid-Open No. 2020-18235
Patent Literature 2: European Patent No. 2578081

### Non-Patent Literature

Non-Patent Literature 1: Negi S, Shigematsu T, Ohya M, et al, Semin Dial.2018
Non-Patent Literature 2: Shinjo H, Maruyama S, et al, Clin Exp Nephrol.2014
Non-Patent Literature 3: K Furuhashi, JASN.2013

### Summary of Invention

### Technical Problem

Conventional cell culture methods using hollow fiber membranes have not always been able to obtain sufficient amounts of cells. This may be due to insufficient cell adhesion to the surface of the hollow fiber membranes.

In view of this situation, an object of the present invention is to provide a method for efficiently culturing MSCs on hollow fiber membranes.

Another object of the present invention is to provide a means for applying cultured MSCs on hollow fiber membranes to diseases.

### Solution to problem

As a result of intensive studies to solve the above problems, the present inventors have found that MSCs are cultured in a state of multiple layers through the use of a hydrophilized hollow fiber membrane and the culturing of MSCs seeded on the outer luminal surface of the hollow fiber membrane under conditions of applying hydrodynamic loading into the hollow fiber membrane. The present inventors have also conceived that the thus cultured MSCs in multiple layers on the hollow fiber membrane are contained in a cartridge so that the MSCs are suitably applicable to therapeutic devices, and thus have completed the present invention.

Specifically, the present invention is as follows.
[1] A method for culturing mesenchymal stem cells (MSCs), including: a step of hydrophilizing the outer luminal surface of a hollow fiber membrane; a step of placing the hydrophilized hollow fiber membrane in a culture solution for seeding MSCs on the outer luminal surface; and a step of culturing MSCs under conditions of applying hydrodynamic loading into the hollow fiber membrane with the cells seeded thereon.
[2] The method according to [1], wherein the culture solution is forced to pass through the lumen of the hollow fiber membrane, so as to apply the hydrodynamic loading into the hollow fiber membrane.
[3] The method according to [1] or [2], wherein the MSCs are adipose-derived MSCs (ASCs).
[4] An MSC culture device, including: a bioreactor that has a hollow fiber membrane having a hydrophilized outer luminal surface; a circulation channel for a culture solution to pass through, including a path that runs through the lumen of the hollow fiber membrane; a culture solution supply section that is connected to one end of the circulation channel for supplying the culture solution to the circulation channel; a collection section that is connected to the other end of the circulation channel for collecting the culture solution discharged from the circulation channel; and a means configured to circulate the culture solution.
[5] A column cartridge containing MSCs and a hollow fiber membrane,
   wherein the MSCs are adhered in multiple layers to the outer luminal surface of the hollow fiber membrane.
[6] The cartridge according to [5], wherein 30% or more of the outer luminal surface of the hollow fiber membrane is coated with one or two or more layers of MSCs.
[7] The cartridge according to [5] or [6], wherein the MSCs are adipose-derived MSCs (ASCs).
[8] A method for producing the cartridge according to any one of [5] to [7], comprising a step of culturing MSCs by the method according to any one of [1] to [3].
[9] A therapeutic device, containing the cartridge according to any one of [5] to [7].
[10] Further, the therapeutic device according to [9], including: a circulation channel for blood or a culture solution to pass through, including a path that runs through the lumen of the hollow fiber membrane within the cartridge; and a means configured to circulate the blood or the culture solution.

### Advantageous Effects of Invention

According to the culture method of the present invention, MSCs can be efficiently cultured in large quantities. In addition, a hollow fiber membrane in which many cultured MSCs are present in multiple layers on the outer luminal surface can be obtained, and such a hollow fiber membrane can be provided in the form of a column cartridge, and thus can be suitably used for treatment that is performed in extracorporeal circulation. The cultured MSCs exhibit the increased production of growth factors, immunosuppressive molecules and EVs, and the decreased production of inflammatory cytokines. Since a large amount of MSCs can be placed on a column cartridge, a large production of growth factors and EVs can be ensured. Therefore, extracorporeal circulation using the column cartridge can efficiently treat various diseases that have been previously expected to be treated with MSC administration, while avoiding the risks associated with cell administration.

### Brief Description of Drawings

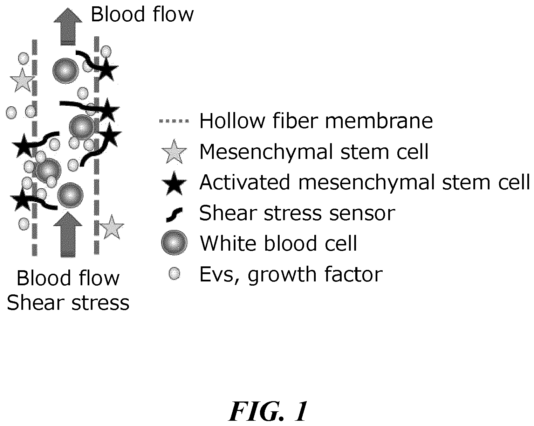

Fig. 1 is a schematic diagram showing a form of the MSC column cartridge wherein MSCs activated by shear stress secrete growth factors/extracellular vesicles (EVs).
Fig. 2 shows micrographs of MSCs cultured on the outer luminal surfaces of hollow fiber membranes. The area sandwiched by A in the micrograph of the example represents the thickness of the cell layers.
Fig. 3 is a fluorescence micrograph showing a shear stress sensor (Arl13b; arrow) expressed on an MSC.
Fig. 4 is a graph showing the expression levels of shear stress sensors expressed on MSCs (black: fluorescent labeling detection with anti-Arl13b antibody, white: control with secondary antibody only).
Fig. 5 shows graphs showing the production of growth factors and inflammatory cytokines in the cultured MSCs. Column-: culture solution in dish culture, Inside: culture solution on the outer luminal side of hollow fiber membrane, Outside: culture solution in culture solution bag outside the hollow fiber membrane cartridge. (*: p<0.01, ANOVA, Tukey's multiple test)
Fig. 6 is a micrograph showing cell membrane components (portions stained with DiD) of MSCs cultured on the outer luminal surface of the hollow fiber membrane.
Fig. 7 shows micrographs showing EVs in MSC culture solutions.
Fig. 8 is a graph showing the particle size distribution of EVs induced by cultured ASCs. (Unit of horizontal axis: nm)
Fig. 9 is a graph showing the concentrations of EVs induced by cultured ASCs in the culture solutions.
Fig. 10 shows graphs showing the expression levels of immunosuppressive molecules in MSCs.
Fig. 11 shows graphs showing the renal function evaluation results (serum creatinine/kidney weight on day 7 after the start of treatment and proteinuria on day 4 after the start of treatment) when the renal dysfunction model rats were subjected to the extracorporeal circulation therapy using MSC (ASC) column cartridges.
Fig. 12 shows micrographs of MSCs cultured on the outer luminal surface of the hollow fiber membranes for 48 hours. (A) Normal culturing, (B) Culturing on hollow fiber membranes not subjected to hydrophilization, seeded cell count 1.0 × 10⁷ cells/100 mL medium, (C) Culturing on hollow fiber membranes subjected to hydrophilization, seeded cell count 0.5 × 10⁷ cells/100 mL medium, (D) Culturing on hollow fiber membranes subjected to hydrophilization, seeded cell count 1.0 × 10⁷ cells/100 mL medium.
Fig. 13 shows graphs showing (A) cell coverage% and (B) the thickness of multiple cell layers of MSCs cultured for 48 hours on the outer luminal surface of the hollow fiber membranes.
Fig. 14 shows graphs showing the renal function evaluation results (serum urea nitrogen, serum creatinine, proteinuria, and kidney weight on day 7 after the start of treatment) when renal dysfunction model rats were subjected to extracorporeal circulation therapy using MSC (ASC) column cartridges. (*: p<0.05, **: p<0.005, ANOVA, Tukey's multiple test)
Fig. 15 is a graph showing the number of ED+1 cells in glomeruli on day 7 after the start of treatment when renal dysfunction model rats were subjected to extracorporeal circulation therapy using MSC (ASC) column cartridges. (**: p<0.005, ***: p<0.001, ANOVA, Tukey's multiple test)
Fig. 16 is a graph showing the survival rate of rhabdomyolysis model rats when the rats were subjected to extracorporeal circulation therapy using MSC (ASC) column cartridges.
Fig. 17 shows graphs showing renal function evaluation results (serum urea nitrogen, serum creatinine, and LDH) when rhabdomyolysis model rats were subjected to extracorporeal circulation therapy using MSC (ASC) column cartridges. (*: p <0.05, Unpaired t test at each day.)

### Description of Embodiments

Next, the present invention is described in detail. However, the present invention is not limited to the following embodiments and can be freely modified within the scope of the present invention.

The culture method according to the present invention is a method for culturing mesenchymal stem cells (MSCs) that includes: a step of hydrophilizing the outer luminal surface of a hollow fiber membrane; a step of placing the hydrophilized hollow fiber membrane in a culture solution for seeding MSCs on the outer luminal surface; and a step of culturing MSCs under conditions of applying hydrodynamic loading into the hollow fiber membrane with the cells seeded thereon.

Hollow fiber membranes are not particularly limited as long as they do not permeate MSCs and can permeate culture solution components and cellular metabolites (waste products).

Examples of the materials include one or two or more types selected from cellulosic materials such as cellulose acetate and recycled cellulose, synthetic materials such as polyethylene, polypropylene, polystyrene, polyvinyl alcohol, polymethyl methacrylate, polyacrylonitrile, fluorinated resin, polyamide, polysulfone, and polyethersulfone and derivatives thereof.

The inner diameter of the hollow fiber membrane preferably ranges from about 200 µm to 500 µm.

The membrane thickness of the hollow fiber membrane may be set within a range where the hollow fiber membrane maintains adequate strength and the thickness does not significantly interfere with the permeability of substances, and is preferably 150 um or less, for example.

The pore size of the hollow fiber membrane is not particularly limited as long as the pores allow the passage of culture solution components such as water, salts, and proteins, while not allowing the passage of cells. However, from the perspective of substance exchange during cell culturing and permeation of useful substances in extracorporeal circulation therapy described below, the hollow fiber membrane desirably has a relatively large pore size for higher efficiency. Specifically, the hollow fiber membrane having a mean pore size of about 0.2 µm to 0.8 µm is preferred. Care must be taken for excessively small pore size, since various biological components may be adsorbed or clogged due to culturing or extracorporeal circulation in such a case.

The shape of a hollow fiber is not particularly limited but is usually circular in cross section. Alternatively, the hollow fiber may be shaped with an irregular cross-section to increase the cell adhesion surface area.

Hydrophilization can be performed by bringing the hollow fiber membrane surface, at least the outer luminal surface, into contact with a hydrophilizing agent. Examples of the hydrophilizing agent include hydrophilic polymers such as polyvinylpyrrolidone, ethylenevinyl alcohol copolymer, hydroxyalkyl cellulose, and vinylpyrrolidone/vinyl acetate copolymer, glycerin, ethanol, and aqueous solutions thereof, and ethanol and aqueous solutions thereof are more preferred.

Hydrophilization of the outer luminal surface improves MSC adhesion to the outer luminal surface of the hollow fiber membrane. In addition, the seeded MSCs first adhere to each other at the sides, so MSCs extend laterally (flat) to form a flat and thinly elongated shape, making them easier to be cultured in multiple layers on the outer luminal surface of the hollow fiber membrane.

In addition, the hydrophilization of the outer luminal surface facilitates the supply of liquid components such as a culture solution and blood during extracorporeal circulation therapy to cultured cells.

**In** addition to the hydrophilization, the outer luminal surface of the hollow fiber membrane may be treated with a coating agent such as collagen or fibronectin to further enhance MSC adhesion.

Cells to be cultured are not particularly limited as long as the cells are MSCs, and examples thereof include bone marrow-derived, adipose-derived, placental-derived, dental pulp-derived cells. Of these, adipose-derived MSCs (ASCs) are particularly preferred from the perspective of application to the treatment of acute kidney injury (AKI).

Animals from which MSCs are derived are not particularly limited, and MSCs may be derived from any animal such as humans, pigs, dogs, mice, and rats.

These cells may contain foreign genes introduced prior to culturing, or may be cells pre-stimulated or pre-processed with other stimulating factors such as antibodies or ligands.

The culture solution is not particularly limited as long as it is normally used for culturing MSCs. For example, DMEM, F12, etc. can be used as the basic medium.

The medium may contain serum. The medium may also be supplemented with, for example, L-glutamine, insulin, transferrin, selenium, fatty acids, thiol glycerol, lipids, nonessential amino acids, vitamins, growth factors, antibiotics, etc., as needed.

Seeding of MSCs onto the outer luminal surface can be performed by adding a cell suspension to the outer luminal surface of the hollow fiber membrane using a syringe or the like. When MSCs are seeded, it is also preferable to keep the pressure inside the lumen of the hollow fiber membrane at a level relatively lower than that outside the lumen for cells to adhere more easily to the hollow fiber membrane. Making the pressure inside the lumen relatively negative can be performed by aspirating the lumen using a syringe or the like, for example.

The number of cells to be seeded is not particularly limited, but can be, for example, 1 × 10³ to 5 × 10⁵ cells/mL relative to the volume of the culture solution. Alternatively, the number of cells to be seeded can be, for example, 1 × 10⁴ to 1 × 10⁶ cells/cm² relative to the outer luminal surface area of the hollow fibers.

In the present invention, the number of cells per hollow fiber can be increased compared to conventional culturing using hollow fiber membranes.

The step of culturing is performed under conditions of applying hydrodynamic loading into the hollow fiber membrane. Hydrodynamic loading is usually applied by circulation, preferably circulating a culture solution through the lumen of the hollow fiber membrane.

In the absence of hydrodynamic loading, MSCs tend to detach from the outer luminal surface due to gravity, but hydrodynamic loading improves the adhesiveness of MSCs existing on the outer luminal surface, making them less likely to detach and much easier to be cultured in multiple layers unless special stress is applied.

Hydrodynamic loading also causes a pressure difference between inside and outside the lumen due to the Bernoulli effect. In addition, the resulting share stress is sensed by the share stress sensors on the MSC surface to activate MSCs, resulting in increased secretion of EVs. Therefore, fluid culturing of hollow fiber membranes enables to culture while causing the production of large amounts of EVs.

In the step of culturing, the flow rate of a culture solution ranges from preferably 2.5 mL/min to 1,460 mL/min. The flow rate may be changed adequately according to the state of cultured cells and the like.

The direction of the circulation of a culture solution may be constant or switched in the opposite direction at appropriate intervals.

Other culture conditions may include a temperature between 33°C and 38°C and a CO₂ concentration between 3% and 5%. The culture time may usually range from 1 to 48 hours, during which time MSCs on the outer luminal surface of the hollow fiber membrane become confluent and MSCs can be easily cultured in one to two layers.

Such a culture method can be performed using a container that can accommodate hollow fiber membranes and a culture solution. More specifically, an MSC culture device is produced, including a bioreactor that has a hollow fiber membrane having a hydrophilized outer luminal surface, a circulation channel for a culture solution to pass through, including a path that runs through the lumen of the hollow fiber membrane, a culture solution supply section connected to one end of the circulation channel to supply the culture solution to the circulation channel, a collection section connected to the other end of the circulation channel to collect the culture solution discharged from the circulation channel, and a means configured to circulate the culture solution.

With such a culture device, MSCs can be cultured more efficiently at a high density per unit volume, and an operation to circulate a culture solution and an operation to supply · collect the culture solution can be simplified.

The bioreactor may have any size and shape as long as it can accommodate hollow fiber membranes and an appropriate amount of a culture solution. The number of hollow fiber membranes is not particularly limited, but the surface area can range from 0.01 m²/60 kg body weight to 1.0 m²/60 kg body weight.

The circulation channel includes the lumen of the hollow fiber membrane in its path and is connected to the bioreactor to circulate the culture solution. Further, a path that runs outside of the hollow fiber membrane may also be included in the circulation paths. The culture solution supply section is connected to one end of the circulation channel and supplies the culture solution to the circulation channel. The collection section is connected to the other end of the circulation channel to collect the culture solution discharged from the circulation channel. Note that the culture solution supply section and the collection section may be combined into a single member for both supply and collection, such as the form of a culture solution bag. The means configured to circulate is used to adjust the direction of circulation, flow rate, velocity, etc., and is not particularly limited, but usually installed to act on the circulation path using pumps, valves, cocks, etc. The culture device may further include members such as a thermostatic bath and a CO₂ incubator.

In a preferred embodiment of the MSC culture device, a culture solution is introduced into the circulation channel from the culture solution supply section, flows through the lumen in the bioreactor connected to the channel, exits the bioreactor, and is then guided through the circulation path to the collection section. A portion or all of the culture solution may be introduced back into the circulation path that is connected to the other end of the bioreactor after leaving the bioreactor, and may be circulated once or multiple times.

With the culture method described above, cultured MSCs are obtained in multiple layers adhered to the outer luminal surface of the hollow fiber membrane.

MSCs cultured in multiple layers on the outer luminal surface of the hollow fiber membrane can be confirmed by microscopic observation or the like. In the present invention, MSCs are present on the outer luminal surface of the hollow fiber membrane, usually in two or more layers, preferably being cultured in one to ten layers. MSCs are present on the outer luminal surface of the hollow fiber membrane in multiple layers having a thickness ranging from preferably 10 µm to 100 µm on the outer luminal surface of the hollow fiber membrane. The number and thickness of such layers are preferably presented over the entire outer luminal surface of the hollow fiber membrane, but not necessarily the entire surface needs to be covered by MSCs as well. For example, preferably 30% or more, more preferably 50% or more, more preferably 80% or more, and most preferably 90% or more of the outer luminal surface of the hollow fiber membrane may be covered by one or two or more layers of MSCs.

Such a hollow fiber membrane with MSCs cultured in multiple layers on the outer luminal surface is preferably applicable to a column cartridge formed of a suitable container containing the hollow fiber membrane accommodated therein.

The number of hollow fiber membranes contained in a cartridge is not particularly limited, but the surface area can range from 0.01 m²/60 kg body weight to 1.0 m²/60 kg body weight, for example.

The number of MSCs in the cartridge is not particularly limited, but can be, for example, 1 × 10⁴ cells/cm² to 1 × 10⁶ cells/cm² relative to the surface area of the hollow fiber membrane.

With MSCs cultured in multiple layers on the outer luminal surface of the hollow fiber membrane, such a column cartridge can accommodate a large amount of MSCs, thus ensuring a large amount of liquid factors such as growth factors secreted by MSCs and EVs production. MSCs remain captured on the outer luminal surface, and secreted fluid factors and EVs can migrate into the lumen through the pores.

Therefore, such a column cartridge can be preferably applied to therapeutic devices for various diseases for which MSC therapy is considered to be effective. Although the therapeutic device is not particularly limited, a form of administering MSC secretions via extracorporeal circulation is preferred, and this can be an excellent therapeutic method in that it allows for efficient treatment while avoiding the risks of administering MSCs themselves into the body.

Examples of various diseases for which MSC therapy is considered to be effective include acute kidney injury (AKI), acute liver injury, acute lung injury, systemic inflammatory response syndrome, and renal injury associated with rhabdomyolysis.

In addition to the column cartridge, the therapeutic device further including: a circulation channel for blood or a culture solution to pass through, including a path that runs through the lumen of a hollow fiber membrane in a cartridge; and a means configured to circulate the blood or the culture solution is preferred for extracorporeal circulation applications. The circulation paths may also include a path that runs outside of the hollow fiber membrane. The means configured to circulate is used to adjust the direction of circulation, flow rate, velocity, etc., and is not particularly limited, but is usually installed to act on the circulation path using pumps, valves, cocks, and the like.

Such embodiments allow useful substances such as growth factors and EVs to be introduced into the body without circulating MSCs themselves.

Moreover, the circulation of blood or a culture solution in a column creates shear stress, the shear stress sensors on the surface of MSCs sense the stress to activate MSCs, thereby promoting the secretion of liquid factors such as growth factors and EVs. Growth factors and EVs act on and specifically induce anti-inflammatory macrophages, making them effective in the treatment of inflammatory diseases such as AKI.

In a preferred embodiment of the therapeutic device, blood or a culture solution flows through the lumen of a hollow fiber membrane in a column cartridge, takes up useful substances such as liquid factors and EVs secreted from MSCs, and is introduced into the body of a patient to be treated via a catheter or the like through the circulation path after leaving the column cartridge. The blood drawn from the patient via the catheter or the like is then introduced back into the column cartridge via the circulation path.

### Examples

The present invention will be described in more detail below using examples, but the present invention is not limited to these examples.

### <Test Example 1> Culturing MSCs on outer luminal surface of hollow fiber membrane

A hollow fiber membrane (outer diameter: 380 µm, inner diameter: 270 µm, membrane thickness: 55 µm, pore size: 0.2 µm, material: polyethylene) was brought into contact with 70% ethanol for 5 minutes to treat the surface, and then washed with HBSS (Ca+, Mg+). The hollow fiber membrane was then placed in a column, and then the outer luminal surface of the hollow fiber membrane was filled with 1 × 10⁷ cells of MSCs (adipose-derived mesenchymal stem cells, prepared by the Department of Nephrology, Nagoya University) while aspirating the lumen of the hollow fiber membrane with a syringe to create negative pressure against the outer luminal part. Cells were cultured for 48 hours under conditions of 37°C and 5% CO₂ while circulating a culture solution (source) through the lumen of the hollow fiber membrane (5 mL/min). Circulation was performed by connecting a culture solution bag and a circulation pump to the column. Cells cultured without circulation of the culture solution were used as a comparative example.

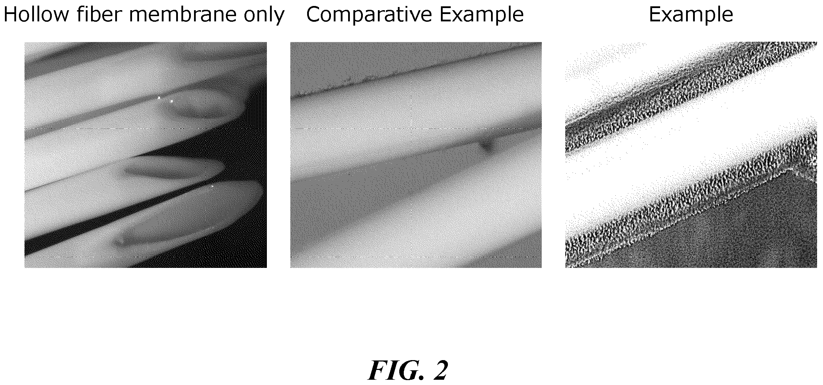

After 48 hours, the hollow fiber membranes were observed. In the comparative example, only a small amount of cells adhered to the hollow fiber membrane, whereas in the example, cells were cultured on the outer luminal surface of the hollow fiber membrane in multiple layers (at least two layers) having almost a uniform thickness, covering the entire hollow fiber membrane (Fig. 2).

### <Test Example 2> Confirmation of expression of shear stress sensor in cultured MSCs

Ten (10) mL of MSC culture medium (prepared by the Department of Nephrology, Nagoya University. See International Publication WO2008/018450 and International Publication WO2011/043136) was added to a culture dish, 1 × 10⁶ cells of MSCs (adipose-derived mesenchymal stem cells, prepared by the Department of Nephrology, Nagoya University) were seeded, and then cultured at 37°C for 48 hours under 5% CO₂ conditions. After 48 hours, MSCs were removed from the culture dish and then stained with an anti-Arl13b antibody (Proteintech). Cells were observed under a fluorescence microscope after staining and fluorescent labeling with the aforementioned antibody was detected by flow cytometry (BD, LSR Fortessa). Arl13b is a ciliated protein, and its presence on MSCs indicates the presence of shear stress sensors.

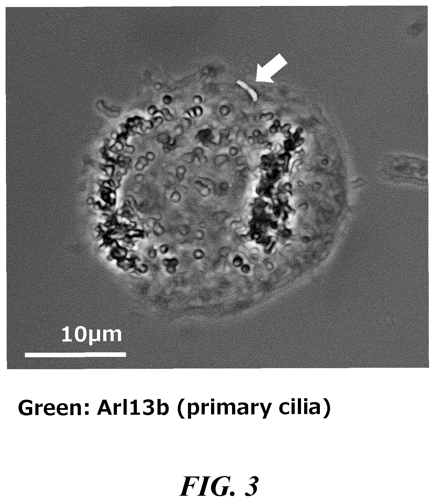

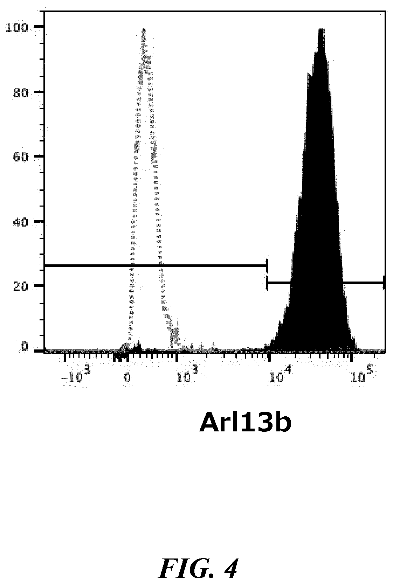

As shown in Figs. 3 and 4, the expression of shear stress sensors was confirmed on MSCs.

### <Test Example 3> Confirmation of production of growth factors and inflammatory cytokines in cultured MSCs

MSCs (adipose-derived mesenchymal stem cells, prepared by the Department of Nephrology, Nagoya University) were seeded 1 × 10⁶ cells per 10 mL of culture solution (prepared by the Department of Nephrology, Nagoya University. See International Publication WO2008/018450 and International Publication WO2011/043136) and then cultured for 48 hours under the same conditions as in Test Example 1 using hollow fiber membranes. MSCs (adipose-derived mesenchymal stem cells, prepared by the Department of Nephrology, Nagoya University) were seeded in a culture dish at 1 × 10⁶ cells per 10 mL of culture solution (prepared by the Department of Nephrology, Nagoya University) and then cultured at 37°C for 48 hours under 5% CO₂ conditions as a comparative example.

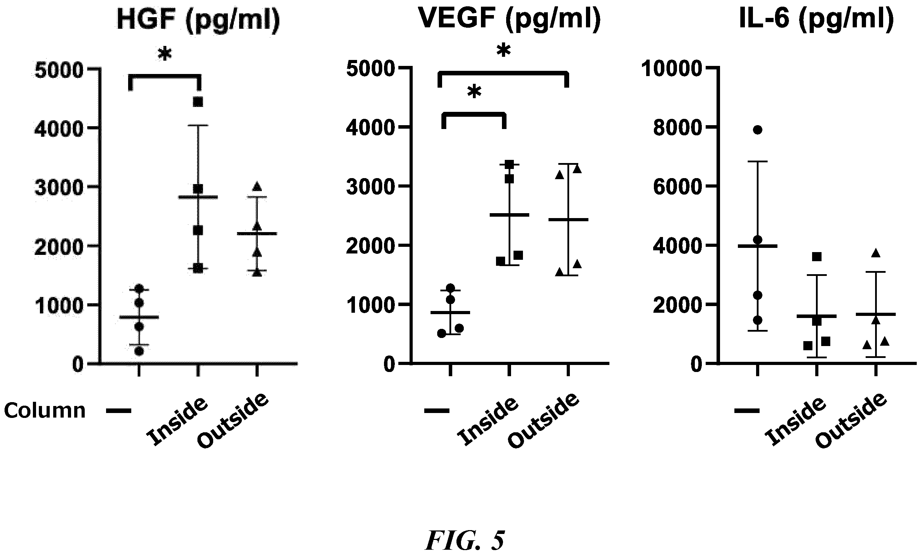

The results are shown in Fig 5. Compared to MSCs cultured in the culture dish, MSCs cultured under circulation conditions on the outer luminal surface of the hollow fiber membrane produced significantly greater amounts of growth factors and smaller amounts of inflammatory cytokines.

### <Test Example 4> Confirmation of induction of EVs production by cultured ASCs

ASCs (adipose-derived mesenchymal stem cells, prepared by the Department of Nephrology, Nagoya University) were cultured in hollow fiber membrane cartridges or culture dishes as in Test Example 3.

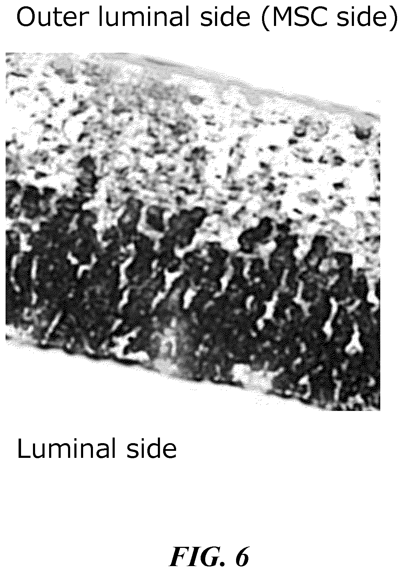

After culturing, the hollow fiber membranes were stained with indocarbocyanine dye (DiD) and observed under a fluorescence microscope. Green fluorescence was detected in the lumen and outside the lumen of the hollow fiber membrane, confirming that cell membrane components secreted from cells had infiltrated from the outer luminal side to the luminal side of the hollow fibers (Fig. 6).

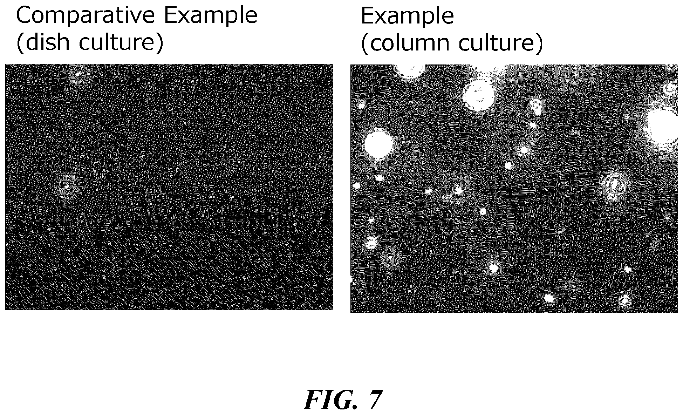

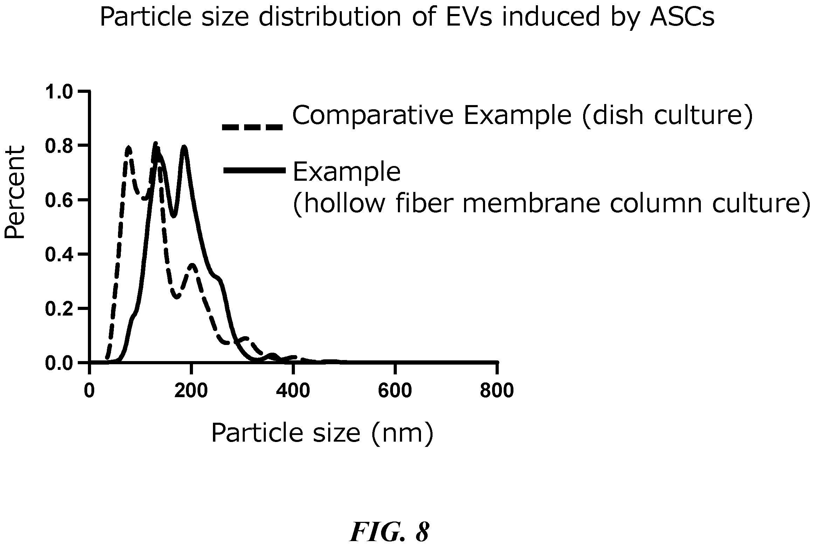

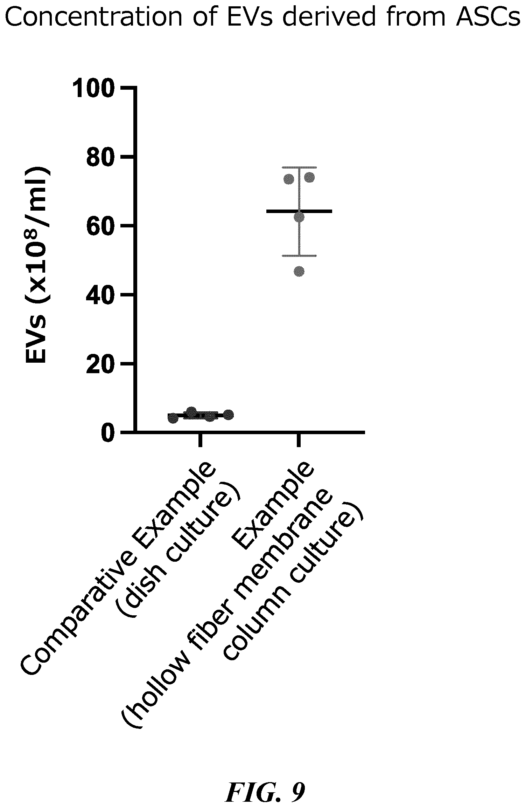

Microscopic examination of the culture solutions after culturing confirmed EVs in both cases, but the particle size and number of EVs were larger and more numerous in MSCs cultured under circulation conditions on the outer luminal surface of the hollow fiber membrane than in MSCs cultured in the culture dish (Fig. 7). When EVs in the culture solution after culturing were detected with a particle size analyzer (Quantum Design, NanoSight), it was quantitatively confirmed that the particle size of EVs was larger (Fig. 8) and the number and concentration of EVs were higher (Fig. 9) in MSCs cultured under circulation conditions on the outer luminal surface of the hollow fiber membrane compared to those cultured in the culture dish.

### <Test Example 5> Confirmation of effect of cultured MSCs on production of immunosuppressive molecules

MSCs (adipose-derived mesenchymal stem cells, prepared by the Department of Nephrology, Nagoya University) were cultured for 48 hours in a hollow fiber membrane cartridge or a culture dish as in Test Example 3. Immunosuppressive molecules (PD1, PDL1, and CD73) of cultured cells were detected by flow cytometry (BD, LSR Fortessa) after culturing.

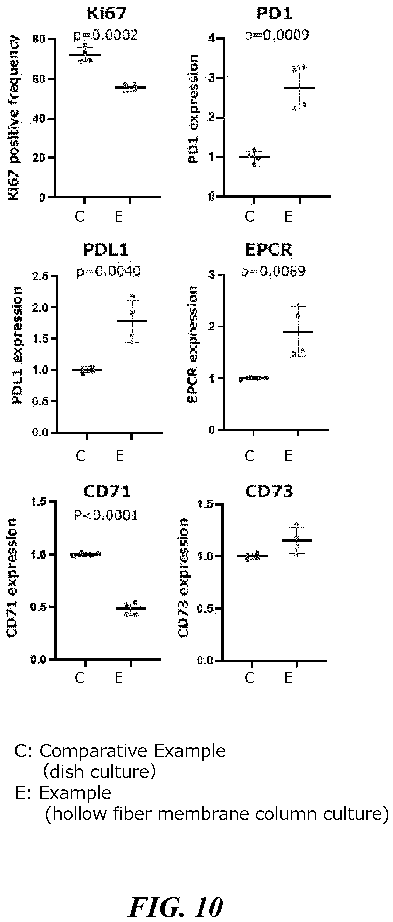

The results are shown in Fig. 10. A significant increase in the production of immunosuppressive molecules was confirmed in MSCs cultured under circulation conditions on the outer luminal surface of the hollow fiber membrane compared to those cultured in the culture dish.

### <Test Example 6> Extracorporeal circulation therapy 1 using ASC column cartridge in renal dysfunction model rats

A catheter was inserted through the right external jugular vein of each WKY rat (9 weeks old, male, W K Y, Charles River Laboratories Japan, Inc. (currently, The Jackson Laboratory Japan, Inc.). Three weeks later, an anti-basement membrane antibody (TF78) was administered at 200 µg/rat to induce renal injury, thereby creating a rat model of acute renal injury (Day 0).

ASCs (adipose-derived mesenchymal stem cells, prepared by the Department of Nephrology, Nagoya University) were seeded at 1 × 10⁶ cells per 10 mL of culture solution (prepared by the Department of Nephrology, Nagoya University) and then cultured using hollow fiber membranes for 48 hours under the same conditions as in Test Example 1. After culturing, a column cartridge containing the hollow fiber membrane was connected to a root tube and a circulation pump, and then connected to the catheter of each model rat above on days 0, 2 and 4 for extracorporeal circulation. Extracorporeal circulation was performed at 1.4 mL/min for 5 hours per session. As a comparison, another rat of the same model was also tested as a control under the same conditions except for the hollow fiber membrane column treatment. Renal functions were evaluated by measuring proteinuria on day 4 and serum creatinine and kidney weight on day 7.

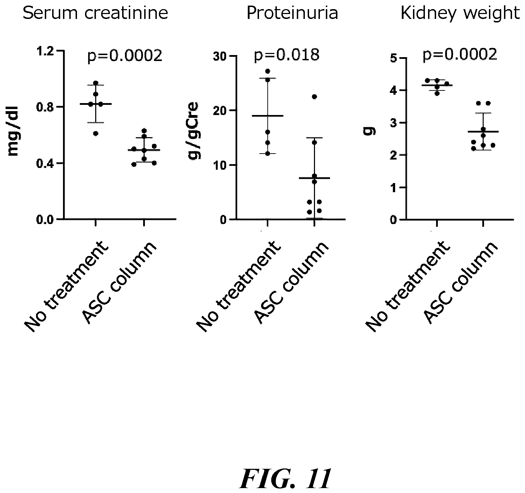

Results are shown in Fig. 11. In all the acute kidney injury model rats, all indices were found to be decreased, confirming a trend toward recovery of renal functions. Regarding the appearance of the kidneys, they were swollen without treatment, but after extracorporeal circulation they were of normal size.

<Test Example 7> Extracorporeal circulation therapy 2 using ASC column cartridge in renal dysfunction model rats

As in Test Example 6, a rat model of acute kidney injury was created (Day 0).

ASCs (adipose-derived mesenchymal stem cells, prepared by the Department of Nephrology, Nagoya University) were cultured for 48 hours under the same conditions as in Test Example 1, using hollow fiber membranes as in Test Example 6. However, the hollow fiber membrane hydrophilized with 70% ethanol before culturing and the one not subjected to hydrophilization were used separately. Cells cultured without circulation of a culture solution were used as a comparative example. The number of cells seeded per 100 mL of culture solution (prepared by the Department of Nephrology, Nagoya University) was 0.5 × 10⁷ cells or 1 × 10⁷ cells.

After culturing, a column cartridge containing each hollow fiber membrane was connected to a root tube and a circulation pump as in Test Example 6, connected to the catheter of each aforementioned model rat on days 0, 2, and 4 for extracorporeal circulation. Extracorporeal circulation was performed at 1.4 mL/min for 5 hours per session. As a comparison, a rat of the same model was also tested as a control under the same conditions except for the hollow fiber membrane column treatment. Renal functions were evaluated by measuring serum urea nitrogen, serum creatinine, proteinuria, kidney weight, and the number of ED+1 cells in the glomeruli on day 7.

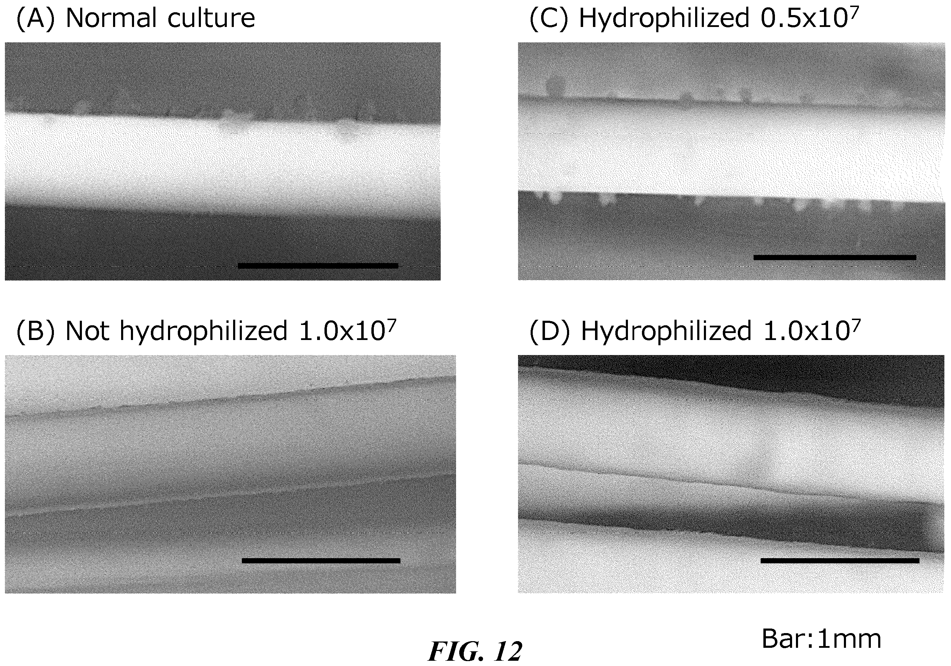

Fig. 12 shows micrographs of hollow fiber membranes observed after 48 hours of culturing. Cells adhered to the outer luminal surfaces of the hollow fiber membranes that had been hydrophilized prior to culturing, especially when cells had been seeded at 1 × 10⁷ cells/100 mL, cells were cultured in multiple layers having an almost uniform thickness, covering the entire hollow fiber membrane. On the other hand, in the comparative example, cells hardly adhered to the hollow fiber membrane.

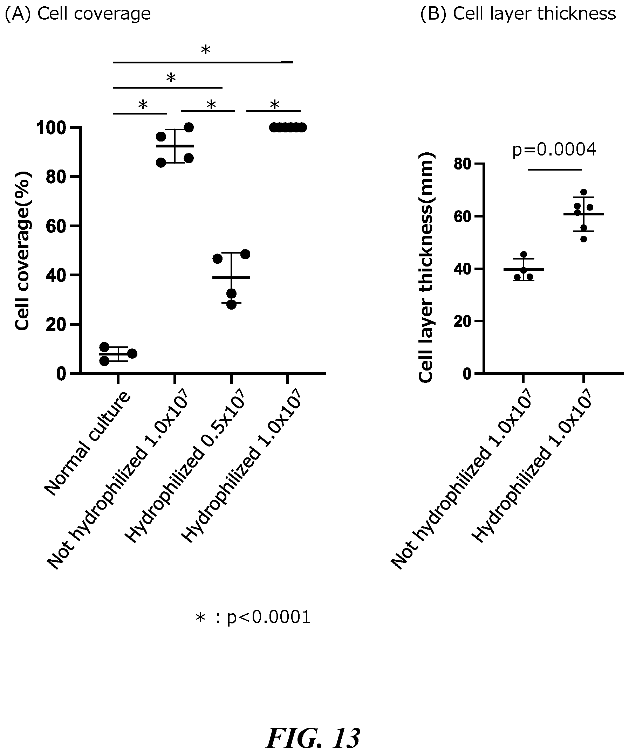

As shown in Fig. 13, hydrophilization and in accordance with the number of cells seeded at the beginning of culturing resulted in greater cell coverage and the thickness of multiple cell layers on the hollow fiber membrane after 48 hours of culturing.

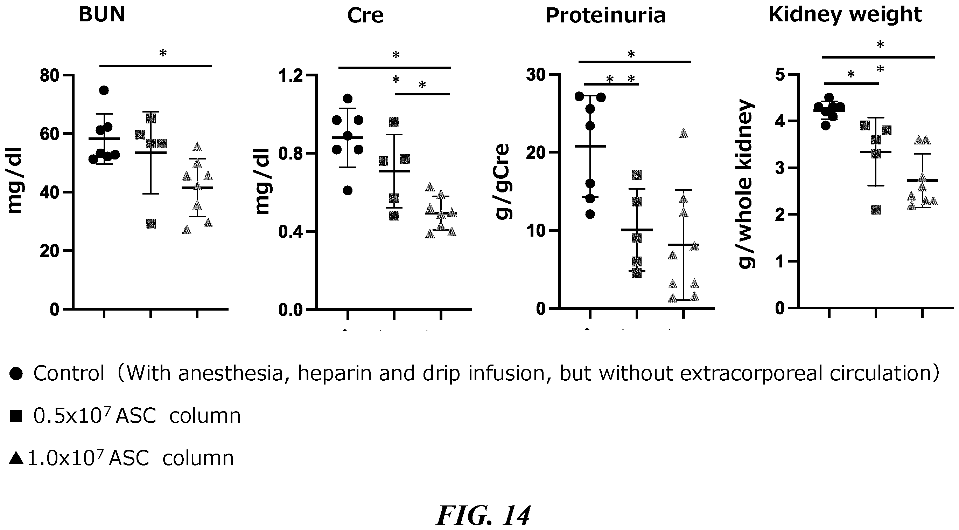

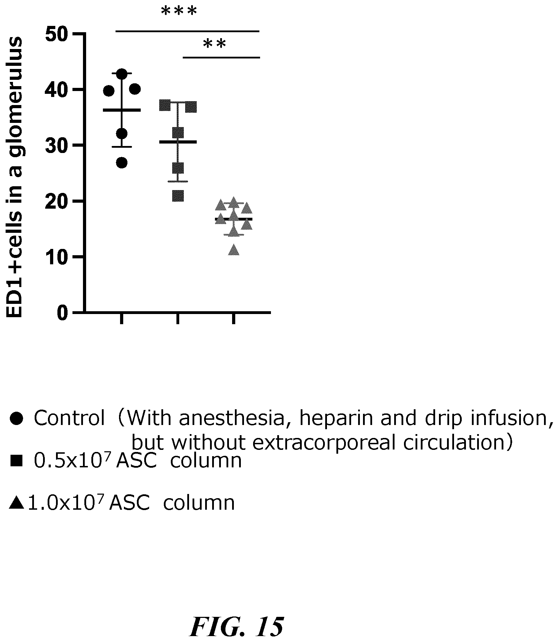

Figs. 14 and 15 show the results of renal function evaluation after treatment with the column cartridge. In acute kidney injury model rats, all indices were found to be decreased, confirming a trend toward recovery of renal functions. Further, a high therapeutic effect was observed in the column cartridges where cells were cultured thick at high coverage% on the hollow fiber membranes.

### <Test Example 8> Extracorporeal circulation therapy using ASC column cartridge in a rat model of rhabdomyolysis induction

A catheter was inserted through the right external jugular vein of each SD rat (10 weeks old, male, Japan SLC, Inc.). Three weeks later, rhabdomyolysis was induced by intramuscular administration of 50% glycerol at 9 mL/kg to create a rat model of renal injury associated with rhabdomyolysis (Day 0).

Each column cartridge of ASCs (adipose-derived mesenchymal stem cells, prepared by the Department of Nephrology, Nagoya University) was prepared using a hollow fiber membrane as in Test Example 6, connected to a root tube and a circulation pump as in Test Example 6, and then connected to the catheter of the aforementioned model rat on Day 0 for extracorporeal circulation. Extracorporeal circulation was performed for 5 hours at 1.4 mL/min per session. As a comparison, an untreated rat of the same model was also tested. Renal functions were evaluated by measuring serum urea nitrogen, serum creatinine, and LDH on days 1, 2, 4, and 5. Survival rates up to day 6 were also measured.

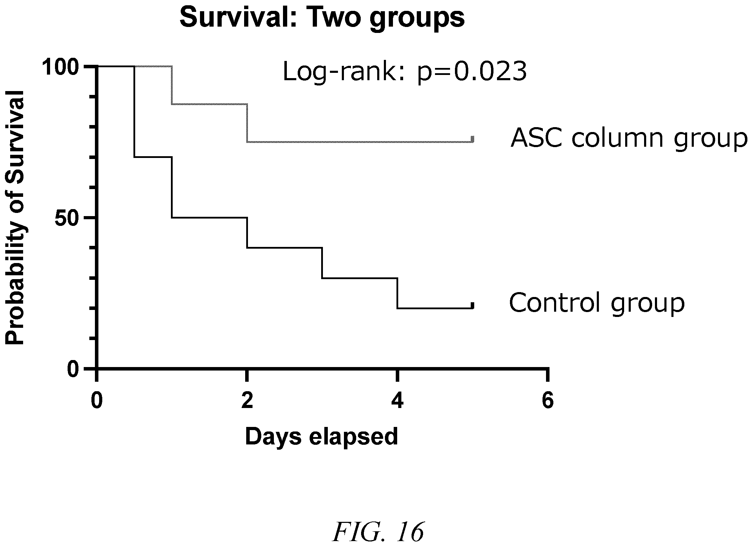

The results of survival rates after rhabdomyolysis induction are shown in Fig. 16. Treatment with extracorporeal circulation using the MSC (ASC) column cartridge improved the survival rate associated with rhabdomyolysis.

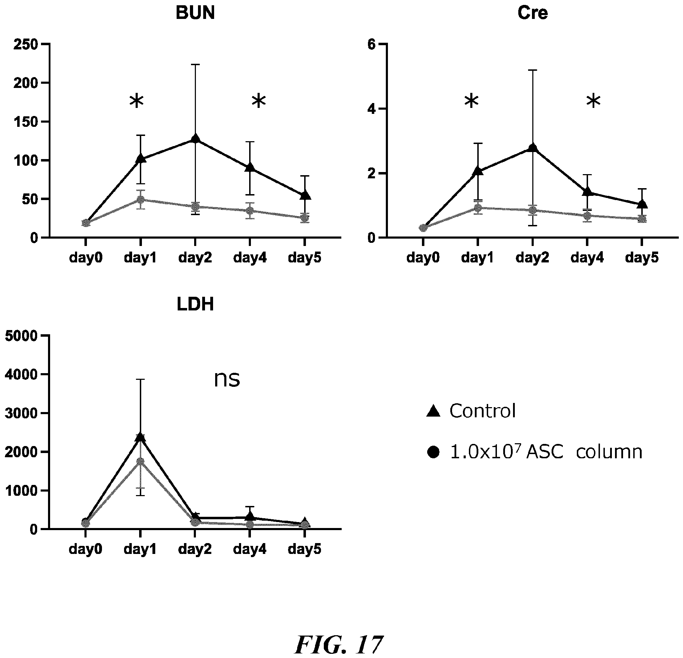

Fig. 17 shows the renal function evaluation results after treatment using the column cartridge. In the model rats, decreases in all indices and restored renal functions were observed, confirming that this treatment is effective against renal damage associated with rhabdomyolysis.

## Claims

1. A method for culturing mesenchymal stem cells (MSCs), comprising:
a step of hydrophilizing the outer luminal surface of a hollow fiber membrane;
a step of placing the hydrophilized hollow fiber membrane in a culture solution for seeding MSCs on the outer luminal surface; and
a step of culturing MSCs under conditions of applying hydrodynamic loading into the hollow fiber membrane with the cells seeded thereon.

2. The method according to claim 1, wherein the culture solution is forced to pass through the lumen of the hollow fiber membrane, so as to apply the hydrodynamic loading into the hollow fiber membrane.

3. The method according to claim 1 or 2, wherein the MSCs are adipose-derived MSCs (ASCs).

4. An MSC culture device, comprising:
a bioreactor that has a hollow fiber membrane having a hydrophilized outer luminal surface; a circulation channel for a culture solution to pass through, including a path that runs through the lumen of the hollow fiber membrane;
a culture solution supply section that is connected to one end of the circulation channel for supplying the culture solution to the circulation channel;
a collection section that is connected to the other end of the circulation channel for collecting the culture solution discharged from the circulation channel; and
a means configured to circulate the culture solution.

5. A column cartridge containing MSCs and a hollow fiber membrane,
wherein the MSCs are adhered in multiple layers to the outer luminal surface of the hollow fiber membrane.

6. The cartridge according to claim 5, wherein 30% or more of the outer luminal surface of the hollow fiber membrane is coated with one or two or more layers of MSCs.

7. The cartridge according to claim 5 or 6, wherein the MSCs are adipose-derived MSCs (ASCs).

8. A method for producing the cartridge according to any one of claims 5 to 7, comprising a step of culturing MSCs by the method according to any one of claims 1 to 3.

9. A therapeutic device, comprising the cartridge according to any one of claims 5 to 7.

10. The therapeutic device according to claim 9, comprising:
a circulation channel for blood or a culture solution to pass through, including a path that runs through the lumen of the hollow fiber membrane within the cartridge; and
a means configured to circulate the culture solution.
